(19) Europäisches Patentamt
European Patent Office
Office européen des brevets

(11) **EP 4 345 841 A1**

(12) **EUROPEAN PATENT APPLICATION**

(43) Date of publication:
**03.04.2024 Bulletin 2024/14**

(21) Application number: **22202812.8**

(22) Date of filing: **20.10.2022**

(51) International Patent Classification (IPC):
**G16H 50/70** (2018.01)   **G16H 20/00** (2018.01)
**G16H 50/30** (2018.01)

(52) Cooperative Patent Classification (CPC):
**G16H 50/70; G16H 20/00; G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.09.2022 US 202263411920 P**

(71) Applicant: **Koninklijke Philips N.V.
5656 AG Eindhoven (NL)**

(72) Inventors:
• **DEN TEULING, Nicolaas Gregorius Petrus**
Eindhoven (NL)
• **GRASSI, Angela**
Eindhoven (NL)
• **SOKORELI, Ioanna**
5656AG Eindhoven (NL)
• **ZHAO, Claire Yunzhu**
5656AG Eindhoven (NL)

(74) Representative: **Philips Intellectual Property &
Standards
High Tech Campus 52
5656 AG Eindhoven (NL)**

(54) **ASSESSING A SUBJECT'S ADHERENCE TO A TREATMENT FOR A CONDITION**

(57)     According to an aspect, there is provided a computer-implemented method (100) for assessing a subject's adherence to a treatment for a condition, the method comprising receiving (102) adherence data indicative of the subject's past adherence to the treatment; receiving (104) medical data indicative of physiological details and a medical history of the subject; determining (106), based on the received adherence data, a non-adherence risk score indicative of a likelihood that the subject will not adhere to the treatment within a defined time period in the future; determining (108), based on the medical data, an adverse event risk score indicative of a likelihood that the subject will experience an adverse medical event; determining (110), based on the non-adherence risk score and the adverse event risk score, a priority classification to be assigned to the subject; and generating (122), based on the priority classification, an instruction signal to be delivered to a recipient

Fig. 1

EP 4 345 841 A1

**Description**

FIELD OF THE INVENTION

The invention relates to adherence management and, more particularly, to assessing how a subject is adhering to a treatment or intervention for a condition, such as a medical condition.

BACKGROUND OF THE INVENTION

Adherence management involves providing support, advice, encouragement and instruction to subjects who are receiving treatment or some other intervention for a condition (e.g., a medical condition). For example, a subject (e.g., a patient) who is receiving positive airway pressure (PAP) therapy to treat a sleep apnea condition may receive adherence management in the form of a live call coaching session, during which the subject receiving the treatment has a real time call with a professional who can educate the subject about the treatment and encourage the subject to complete the treatment. Such real time coaching can be particularly effective, but is costly and time-intensive for care providers.

It has been found that subjects with comorbidities tend to have a greater rate of negative health outcomes, readmission and/or mortality compared to subjects with only one condition, and this may be due to one of the conditions affecting the subject's ability to adhere to the treatment for the other condition. Furthermore, inadequately or insufficiently treated sleep apnea may worsen other conditions from which the subject is suffering. Therefore, it is desirable to have a system that can help to improve a subject's adherence to a treatment or intervention, particularly for a subject with comorbidities. It is also desirable to have a system that is able to assign adherence management resources in a more time-efficient and cost-efficient manner.

SUMMARY OF THE INVENTION

In order to mitigate or overcome at least some of the above-identify problems, there is a desire to prioritize subjects in need of adherence management according to how likely the subjects are to adhere to the treatment, and also according to how at risk they are of experiencing an adverse medical event. The inventors of the present disclosure have realized that, based on a subject's past adherence to a particular treatment, and details of their medical history, it is possible to generate a score that can be used to prioritize the subject among a population of other subjects who would benefit from adherence management. In this way, adherence management can be provided to those who are deemed to be highest priority, which may for example be those subjects who are least likely to adhere to the treatment and/or who are most likely to suffer an adverse medical event.

According to a first specific aspect, there is provided a computer-implemented method for assessing a subject's adherence to a treatment for a condition, the method comprising: receiving adherence data indicative of the subject's past adherence to the treatment; receiving medical data indicative of physiological details and a medical history of the subject; determining, based on the received adherence data, a non-adherence risk score indicative of a likelihood that the subject will not adhere to the treatment within a defined time period in the future; determining, based on the medical data, an adverse event risk score indicative of a likelihood that the subject will experience an adverse medical event; determining, based on the non-adherence risk score and the adverse event risk score, a priority classification to be assigned to the subject; and generating, based on the priority classification, an instruction signal to be delivered to a recipient.

By determining a priority classification (e.g., a score) for the subject based on the non-adherence risk score and the adverse event risk score for a subject, it is possible to take appropriate action given that subjects priority classification. In this way, a subject who is suffering from comorbidities, and who is receiving treatment for one of those conditions, may be rightly prioritized for adherence management for the treatment of a subject who is suffering from just one condition and may therefore be less at risk of experiencing an adverse medical event. Similarly, valuable adverse management resources may be provided for higher-priority subjects, rather than those subjects who are deemed to be lower-priority.

In some embodiments, generating the instruction signal may comprise generating a signal to instruct the recipient to take action to improve the subject's adherence to the treatment.

The signal may comprise at least one of: a signal to instruct an operator to arrange a coaching session with the subject; a signal to instruct an operator to initiate contact with the subject; a signal to instruct a computing device to initiate contact with the subject; a signal to adjust an operating parameter of a treatment device.

The adherence data may, in some embodiments, comprise data selected from a group comprising: an indication of an average adherence to the treatment over a defined period; an indication of a number of days in a defined period when the subject did not adhere to the treatment; and an indication of a variability of adherence to the treatment over a defined period.

The medical data may comprise data selected from a group comprising: an age of the subject, a weight of the subject, a blood pressure of the subject, a heart rate of the subject, a breath rate of the subject, a comorbidity associated with the subject and an indication of the presence of one or more biomarkers in the subject, the biomarkers selected from a group comprising: creatinine kinase, C-reactive protein, B-type natriuretic peptide (BNP), troponin I, troponin T, and N-terminal fragment of proBNP (NT-proBNP).

In some embodiments, the computer-implemented method may further comprise receiving covariate data indicative of one or more covariates that affect the health of the subject. The step of determining a priority classification to be assigned to the subject may further be based on the covariate data.

The covariate data may comprise data selected from a group comprising: an indication of an expected future adherence to the treatment, an age of the subject, a gender of the subject, a race of the subject, a socio-economic class of the subject, an indication of comorbidities suffered by the subject, a blood oxygen level of the subject, and an indication of an apnea or hypopnea suffered by the subject.

In some embodiments, determining the priority classification may comprise applying the non-adherence risk score and the adverse event risk score to a model of the form:

$$f(R_{\text{prio}}) = g(R, Z | \beta)$$

where $f(R_{prio})$ is a priority transformation function; $R$ is a vector of the non-adherence risk score, $R_{NA}$, and the adverse event risk score, $R_{AE}$; $Z$ is a vector of covariates that affect the health of the subject; $\beta$ is a vector of model parameters; and g is a function that calculates a score based on $R$ and $Z$ given $\beta$.

The computer-implemented method may, in some embodiments, further comprise receiving, via a user input, an indication of an adjustment to be made to at least one parameter in the vector of model parameters. The step of determining a priority classification may be based on the adjusted vector of model parameters.

Determining the priority classification may comprise applying the non-adherence risk score and the adverse event risk score to a trained predictive model. In some embodiments, the trained predictive model comprises a Cox proportional-hazards model.

The computer-implemented method may further comprise applying weights to the non-adherence risk score and the adverse event risk score. The step of determining a priority classification may be based on the weighted non-adherence risk score and the weighted adverse event risk score.

The computer-implemented method may, in some embodiments, further comprise receiving medical condition data relating to a particular medical condition from which the subject is suffering. The method may further comprise determining, based on the medical condition data, a medical condition risk score indicative of a likelihood that the subject will suffer an adverse event related to the medical condition. The step of determining a priority classification may be further based on the medical condition risk score.

According to a second specific aspect, there is provided an apparatus comprising a processor configured to perform steps of the method disclosed herein.

According to a third specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform steps of the method disclosed herein.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a flowchart of an example of a method for assessing a subject's adherence to a treatment for a condition;
Fig. 2 is a schematic illustration of an example of an apparatus for assessing a subject's adherence to a treatment for a condition; and
Fig. 3 is a schematic illustration of an example of a processor in communication with a computer-readable medium.

DETAILED DESCRIPTION OF EMBODIMENTS

Helping patients to adhere to a treatment regime for a particular condition (e.g., medical condition) can be particularly important, especially if non-adherence to the treatment can lead to a worsening of the condition. Patients who suffer from multiple conditions (i.e., comorbidities) may be at a particular disadvantage if the treatment for one of the conditions is stopped or if the condition is not treated as intended. For example, a patient suffering from heart failure as well as sleep-disordered breathing (HF-SDB) may have an increased likelihood of readmission to a medical facility or mortality, compared to a patient suffering just from heart failure. One treatment for SDB is positive airway pressure (PAP) therapy, and it has been shown that adherence to PAP therapy significantly reduces the likelihood of readmission and mortality. More particularly, it has been shown that patients receiving daily PAP therapy for a defined minimum duration (e.g., at least 4 hours) have a reduced likelihood of readmission and/or mortality.

Different types of adherence management may be used to assist a subject with adhering to a treatment. Adherence management techniques may, for example, range from simply sending a message to the subject to remind them to use a treatment device, to initiating a live coaching session between a professional and the subject. According to the present disclosure, the nature of the adherence management that is used depends on various features of the subject, which are used to prioritize the subject among other people who would benefit from adherence management.

Embodiments disclosed herein provide a mechanism by which a subject can be prioritized based on various factors, including their past adherence to the treatment, and their medical history. Based on these factors, a set of scores can be determined which are used to determine a priority level or classification for the subject and based on the priority classification, and appropriate action can be taken. In this way, subjects (e.g., patients) can be prioritized based on an expected patient outcome and on the likelihood of therapy adherence. Due to the emphasis on patient outcome, the present invention is applicable in the context of patients suffering from sleep-disordered breathing (SDB) with one or more comorbidities, for example HF-SDB. The patient suffering from SDB may, for example, be receiving PAP therapy, or continuous positive airway pressure (CPAP) therapy, in order to treat the SDB condition. If the patient is also suffering from a comorbidity, then it may be more important that they continue the course of treatment for the SDB to reduce the risk that one of the conditions makes the other condition significantly worse.

Prioritizing subjects using the disclosed techniques means that those subjects who are expected to benefit the most from improved adherence may be prioritized higher than those who are less likely to benefit, and these techniques also enable subjects to receive a lower priority score if they are considered to have a lower risk of suffering an adverse medical event, thereby enabling healthcare providers to offer a more cost-effective service.

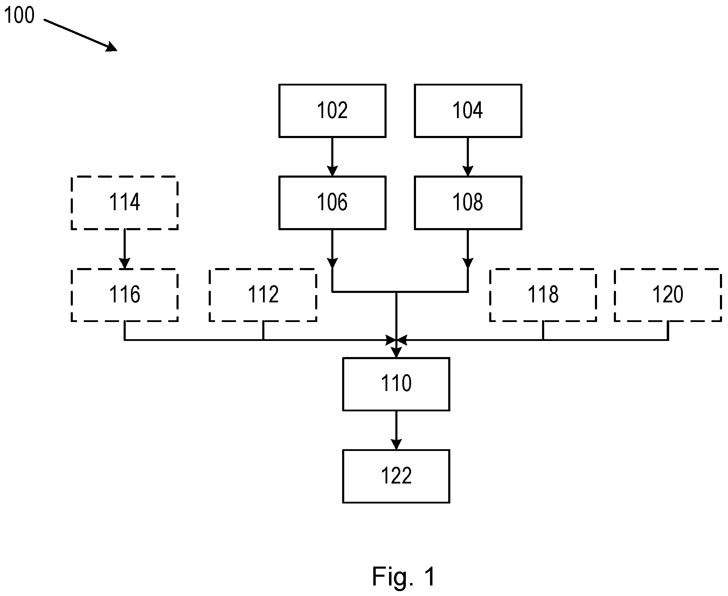

Referring now to the drawings, Fig. 1 is a flowchart of an example of a method 100 for assessing a subject's adherence to treatment for a condition. The method 100 may, for example, comprise a computer-implemented method, which may be performed using one or more processors. The method 100 comprises, at step 102, receiving adherence data indicative of the subject's past adherence to the treatment. The adherence data provides an indication of how well the subject has adhered to treatments for a condition in the past. In some examples, the adherence data may relate to a particular duration, such as the last 6 months, the last 30 days, the last 14 days, the last 24 hours, or the like, and this may depend on the condition being treated and/or the treatment being provided.

The adherence data may comprise data from one or more sources, relating to one or more aspects of the subject's adherence history. The adherence data may comprise an indication of an average adherence to the treatment over a defined period, such as the past week, 2 weeks, or the like. In some examples, the adherence data may comprise an indication of a number of days in a defined period when the subject did not adhere to the treatment. For example, for a subject receiving PAP therapy to treat up sleep-disordered breathing, then the adherence data may include an indication of the number of days in the past 2 weeks when the subject failed to administer the PAP therapy for the minimum required duration. The adherence data may comprise an indication of a variability of adherence to the treatment over a defined period, such as an indication of how the subject varied the application of a particular treatment over the past 2 weeks.

In some embodiments, the adherence data may include other data, such as data indicative of the psychological aspects. For example, data indicative of the subject's motivation to treat the condition, the subject's self-efficacy in relation to the treatment, and/or the importance to the subject of treating the condition. Non-behavioral aspects of the treatment may also be taken into account and included in the adherence data, including an indication of the severity of the condition, an indication of the symptoms suffered by the subject, an indication of whether the subject notices the benefits of the treatment, and an indication of the treatment comfort for the subject. In the case of PAP therapy, the treatment comfort may be determined using a measurement of PAP therapy mask leakage, for example. In other examples, the adherence data may include data indicative of external factors, such as a level of support and/or education that the subject has previously received. One or more of these aspects may be taken into account when determining the likely response of the subject to receiving adherence management.

The adherence data may be obtained from a storage device (e.g., a database containing data related to the subject, stored on a memory), obtained from one or more sensors or devices, such as treatment devices used by the subject, and/or entered manually by a user (e.g., the subject or a medical professional).

At block 104, the method 100 comprises receiving medical data indicative of physiological details and a medical history of the subject. Physiological details of the subject and the subject's medical history may provide an indication of any medical conditions that the subject is currently experiencing or suffering from, along with medical conditions that the subject has suffered from in the past. Along with physiological details of the subject and other medical data relating to the subject, this can provide a good overview of the health of the subject and a likelihood that the subject will become ill or require further medical assistance in the near future.

The medical data may comprise data selected from a group comprising: an age of the subject, a weight of the subject, a blood pressure of the subject, a heart rate of the subject, a breath rate of the subject. The weight, blood pressure, heart rate and/or breath rate may be obtained from recently-obtained measurements, may be newly measured, for example in response to a prompt or request provided to the subject, and/or may be entered manually by the subject or another user the medical data may include an indication of a comorbidity associated with the subject, such as an indication that the subject is suffering from heart failure and sleep-disordered breathing. In some embodiments, the medical data may comprise an indication of the presence of one or more biomarkers in the subject. Such biomarkers may include biomarkers selected from a group comprising: creatinine kinase, C-reactive protein, B-type natriuretic peptide (BNP), troponin I, troponin T, and N-terminal fragment of proBNP (NT-proBNP). The presence of such biomarkers may provide an indication that the subject is suffering from a particular medical condition or is at risk of suffering from a medical condition.

The adherence data and the medical data may then be used to determine risk scores. The method 100 comprises, at step 106, determining, based on the received adherence data, a non-adherence risk score, $R_{NA}$, indicative of a likelihood that the subject will not adhere to the treatment within a defined time period in the future. The defined time period may be selected depending on the condition and/or the treatment, but may for example be one day, one week, 2 weeks, 1 month, or the like. The non-adherence risk score can thus provide an indication of how likely or unlikely it is that the subject will adhere to the treatment.

The non-adherence risk score may be determined (e.g., calculated) using a predictive model or algorithm, which is capable of (e.g., trained to) determine the non-adherence risk score using inputs based on the adherence data discussed herein. In some embodiments, the predictive model may comprise a logistic regression model, a random forest classifier, and/or one or more other supervised learning models or algorithms. The output of the predictive model (i.e., the non-adherence risk score) may be of arbitrary range, but may in general be associated with the probability of future non-adherence to the treatment by the subject. In other words, a subject with a higher non-adherence risk score is more likely to become non-adherent to the treatment than a patient with a lower non-adherence risk score.

At step 108, the method 100 comprises determining, based on the medical data, an adverse event risk score, $R_{AE}$, indicative of a likelihood that the subject will experience an adverse medical event. In some examples, and adverse event may comprise readmission to a medical facility, such as a hospital, or some other adverse medical event that may lead to the subject requiring medical assistance. In some examples, the adverse event may comprise mortality. The adverse event risk score may, in some examples, indicate a likelihood that the subject will experience an adverse medical event within a defined time period, such as 30 days, and the time period may be determined based on the method or adverse event risk score.

The adverse event risk score may, in some embodiments, be determined (e.g., calculated using a predictive model or algorithm, which is capable of (e.g., trained to) determine the adverse event risk score using inputs based on the medical data discussed herein. In some embodiments, the predictive model may comprise a logistic regression model, a random forest classifier, and/or one or more other supervised learning models or algorithms. The model used to determine the adverse event risk score may use any medical data for the subject available up to the date or moment that the model is used. In other examples, the model may be based on a baseline risk model computed at the time that the subject was last discharged from a medical facility.

The method 100 comprises, at step 110, determining, based on the non-adherence risk score and the adverse event risk score, a priority classification to be assigned to the subject. The priority classification is determined (e.g., calculated) effectively by weighing up the non-adherence risk score together with the adverse event risk score. In practice, the priority classification, which may take the form of a score, such as a value or a percentage, may be determined using a model or algorithm, discussed in greater detail below. Determining the priority classification (step 110) may comprise applying the non-adherent risk score and the adverse event risk score to a trained predictive model. Examples of predictive models that may be used are discussed in greater detail below. In some examples, however, the trained predictive model may comprise a machine learning model (e.g., an artificial neural network model), a logistic regression model or a Cox proportional-hazards model, for example.

In some embodiments, the priority classification may be calculated based only on the non-adherence risk score and the adverse event risk score. In other embodiments, however, other data relevant to (e.g., that may affect) the health outcome of the subject may also be taken into account. The other relevant data is referred to herein as covariate data, and this may alternatively be referred to as data relating to additional relevant factors, explanatory variables, or factors affecting or associated with the health of the subject. At step 112, the method 100 may further comprise receiving covariate data indicative of one or more covariates that affect the health of the subject. The covariate data may include any additional data relating to the subject that could affect the health of the subject and which could therefore be taken into account when determining the priority classification.

The covariate data may for example comprise an indication of an expected future adherence to the treatment. Such data may for example be relevant if the subject has received recent coaching or education about the importance of adhering to the treatment. In some examples, the covariate data may include an age of the subject, a gender of the subject, a race of the subject and/or a socio-economic class of the subject, as these might provide an indication of a likely health outcome given other subjects of a similar age, gender, race and/or socio-economic class. The covariate data may, in some embodiments, comprise more medical data relating to the subject, such as an indication of comorbidities suffered by the subject, a blood oxygen level of the subject, and/or an indication of an apnea or hypopnea suffered by the subject. One or more of the above examples might be combined with the adherence data and the medical data to result in an even more valuable priority classification.

In examples where covariate data is provided, determining a priority classification to be assigned to the subject may be further based on the covariate data. For example, the covariate data may be provided as an input to the predictive model, along with the non-adherence risk score and the adverse event risk score.

According to some embodiments, the present invention may be used to calculate a priority classification based on a specific medical condition or medical conditions from which the subject is suffering, and the priority classification determined in such cases may be tailored according to the medical condition(s), if data relating to the medical condition(s) is available.

In such examples, the method 100 may further comprise, at step 114, receiving medical condition data relating to a particular medical condition from which the subject is suffering. Examples of such medical conditions include cardiovascular diseases, hypertension, chronic obstructive pulmonary disease (COPD) or diabetes, and the nature of the medical condition data received and used may depend on the medical condition. For example, if the subject is suffering from one or more cardiovascular diseases, the relevant medical condition data may include the age of the subject, the gender of the subject, the race of the subject, the socio-economic status of the subject, results of a physical examination of the subject (e.g., details of edema or jugular vein distension), a heart rate of the subject, a blood pressure of the subject, biomarkers present in the subject (e.g., circulating natriuretic peptide levels) a weight of the subject, body mass index (BMI) of the subject and/or an activity level of the subject. If the subject is suffering from hypertension, the relevant medical condition data may include the age of the subject, the gender of the subject, a blood pressure of the subject, a heart rate of the subject, an indication of a type of hypertension suffered by the subject, a weight of the subject and/or a BMI of the subject. If the subject is suffering from COPD, the relevant medical condition data may include an age of the subject, a gender of the subject, spirometry grades of the subject, an indication of whether or not the subject smokes, an indication of the number of years since the subject last to suffer from COPD, an indication of whether or not the subject has a cough, an indication of whether or not the subject is producing sputum, an indication of whether or not the subject is suffering from dyspnea, an indication of an exasperation level, and a history of this level for the subject (e.g., moderate or severe), an indication of the COPD gold grade, a weight of the subject, a BMI of the subject, and/or an indication of comorbidities suffered by the subject. If the subject is suffering from diabetes, then the relevant medical condition data may include an age of the subject, I gender of the subject, a weight of the subject, a BMI of the subject, an indication of the blood glucose level of the subject, an indication of whether or not the subject smokes, and/or an indication of an activity level of the subject.

At step 116, the method 100 may comprise determining, based on the medical condition data, a medical condition risk score, $R_{MC}$, indicative of a likelihood that the subject will suffer an adverse event related to the medical condition. The medical condition risk score may be determined in a manner similar to how the other risk scores discussed herein are determined, for example using a model such as a predictive model.

In examples in which a medical condition risk score has been determined, the step of determining a priority classification may be further based on the medical condition risk score. In other words, the step of determining a priority classification (step 110) may be based on the non-adherence risk score, the adverse event risk score and the medical condition risk score.

In one example, determining the priority classification may comprise applying the non-adherence risk score and the adverse event risk score to a model of the form:

$$f\left(R_{\mathrm{prio}}\right) = g(R, Z|\beta) \qquad [1]$$

where $f(R_{prio})$ is a priority transformation function; $R$ is a vector of the non-adherence risk score, $R_{NA}$, and the adverse event risk score, $R_{AE}$; $Z$ is a vector of covariates that affect the health of the subject; $\beta$ is a vector of model parameters; and $g$ is a function that calculates a score based on $R$ and $Z$ given $\beta$.

In examples in which a medical condition risk score is also determined, the vector $R$ may also include $R_{MC}$. In examples in which covariate data is not provided or available, then the vector $Z$ may be omitted from the model shown above.

The model parameters including the vector $\beta$ may, for example, comprise respective weights of parameters or inputs (e.g., respective weights in a linear combination of inputs provided to a predictive model). The weights may be selected based on domain knowledge in the relevant field (e.g., literature) and/or may be adjusted manually. In some examples, the model parameters may comprise weights applied to one or more of the risk scores (e.g., the non-adherents risk score, the event risk score and/or the medical condition risk score). For example, the method 100 may comprise, at step 118, applying weights to the non-adherent risk score and the adverse event risk score (and the medical event risk score when used). When weights have been applied to the risk scores, the step of determining a priority classification (e.g., step 110) may be based on the weighted non-adherents risk score and the weighted adverse event risk score (and the weighted medical event risk score when used).

Parameters included in the vector of model parameters and/or the weights applied to such parameters may be adjusted by a user in order to improve the output of the predictive model determining the priority classification. In some embodiments, the method 100 may comprise, at step 120, receiving, via a user input, an indication of an adjustment to be made to at least one parameter in the vector of model parameters. In examples in which an adjustment is made to one or more parameters, the step of determining a priority classification (step 110) may be based on the adjusted vector of model parameters.

If a training dataset is available, then a model used to determine the priority classification may be trained and/or calibrated based on data specific to a particular subject, such as data relating to an adverse medical event that the subject is at risk from. In some examples, a survival analysis of model may be used to determine a likelihood (e.g., risk) that a particular subject will experience/suffer from the adverse medical event. One example of a survival analysis model is a Cox proportional-hazards model, and this may be used to determine the priority classification. In an example of the Cox proportional-hazards model, an expected hazard rate, $R_{prio}$, of the subject experiencing an adverse medical event that is intended to be delayed or prevented by means of adherents to the treatment may take the form:

$$R_{prio} = h(t) = h_0(t)\exp[\beta_0 R_{NA} + \beta_1 R_{AE} + Z\gamma] \qquad [2]$$

where $Z$ is a vector of inputs including, for example, a mean level of the subject's previous adherents to the treatment, and expected future level of adherents to the treatment, the subject's age, gender, race, socio-economic class and/or weight, comorbidities of the subject, cardiac characteristics and/or respiratory characteristics of the subject, and/or any relevant biomarkers present in the subject. In the model [2] above, $h(t)$ is an estimated patient hazard at time $t$ (e.g., an estimate of how the risk of event occurrence changes with time), $h_0(t)$ is a baseline population hazard at time $t$, $\beta_0$ and $\beta_1$ are effect parameters, and $\gamma$ is a vector of model parameters, such as weights to be applied to the inputs included in the vector $Z$.

The Cox proportional-hazards model, such as the model [2] above, determines a likelihood that the subject will suffer from an adverse medical event, based on a likelihood of a given population of the people, and tailoring that likelihood specifically for the subject. Such a model may provide a value as its output, and this value can be compared to values for other people in order to prioritize people accordingly.

Once the priority classification has been determined (step 110), the method 100 may further comprise, at step 122, generating, based on the priority classification, an instruction signal to be delivered to a recipient. The instruction signal may be delivered to a recipient device, such as a computer, workstation, smart phone, or the like, to provide information (e.g., determined priority classification) to a user of the recipient device, or the instruction signal may comprise a signal to operate a device based on the priority classification. In some embodiments, generating the instruction signal may comprise generating a signal to instruct the recipient to take action to improve the subject's adherence to the treatment. For example, if the determined priority classification shows that the subject is particularly high priority, then the generated signal may cause or instruct urgent action to be taken compared to if the priority classification shows that the subject is relatively low priority. The threshold for a "high priority" subject may be selected as a defined proportion of eligible subjects (e.g., the top 30% of all subjects ranked in order of priority may be classed as high priority), or as a function of the available capacity and resources of an adherence management service. More generally, the function $f$ in model [1] above may comprise a stratification function that categorizes a subject according to a defined set of categories. In another embodiment, a trained clustering model or algorithm may be used to group similar subjects based on the determined risk scores, the determined priority classifications, and/or other factors. Such clustering may enable subjects to be clustered or stratified (e.g., classified into groups or priority levels) based on the various data available.

The signal generated at step 122 may comprise at least one of: a signal to instruct an operator to arrange a coaching session with the subject; a signal to instruct an operator to initiate contact with the subject; a signal to instruct a computing device to initiate contact with the subject; a signal to adjust an operating parameter of a treatment device. The nature of the signal generated may depend on the determined priority classification. For example, for a high priority subject, a coaching session may be arranged quickly while, for a low priority subject, the signal may instruct an operator to contact the subject within a few weeks.

A subject determined to have a medium priority may be provided with the same adherence management support as a high priority subject, but over a longer timescale (e.g., not as urgently). In some examples, where resources allow, a medium priority subject may be treated in a manner similar to a high priority subject. The patient determined to be low priority may be assigned a more scalable/economic form of adherence management support including, for example, the provision of support material via email or via a smart phone application. In such cases, the instruction signal generated at step 122 may comprise an instruction to initiate automated communication with the subject.

Various modifications and/or additions may be made to the method 100 discussed herein. For example, steps of the method 100 may be performed in an audit different to that presented herein. Additional risk scores may be determined, based on other available data relating to the subject, and these may be used in the determination of the priority classification. Based on the determined priority classification, subjects may be classified within a defined group (e.g., high priority, medium priority, low priority, and the like) and/or assigned a priority score. Weights applied to the various risk scores may be calibrated or optimized by training a logistic regression model on data relating to subjects for whom it has been determined that urgent adherence management is required. The model used to determine the priority classification may be trained using various data, including for example an expected time until the adverse medical event is likely to occur, and/or reference data relating to priority scores for subjects obtained from an existing adherence management system. In some examples, the determined priority classification may be adjusted or modified based on an indication of the last time that the subject was contacted regarding adherence management.

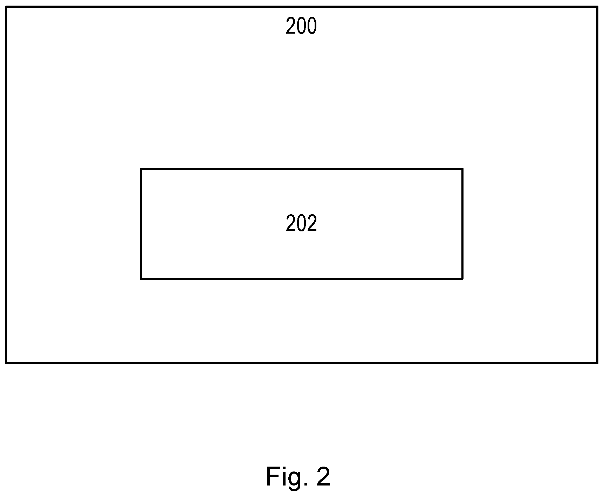

Embodiments of the present invention also provide an apparatus, such as an apparatus for assessing a subject's adherence to a treatment for a condition. Fig. 2 is a schematic illustration of an example of an apparatus 200. The apparatus 200 comprises a processor 202 configured to perform steps of the method 100 disclosed herein. In some examples, multiple processors may be used to perform steps of the method. For example, a different processor may perform each of the steps of the method 100. While the processors may, in some embodiments, be located within the same apparatus or device (e.g., the apparatus 200), in other embodiments, the processors may be located remotely from one another, such as in a cloud-computing environment, and may be configured to communicate with one another.

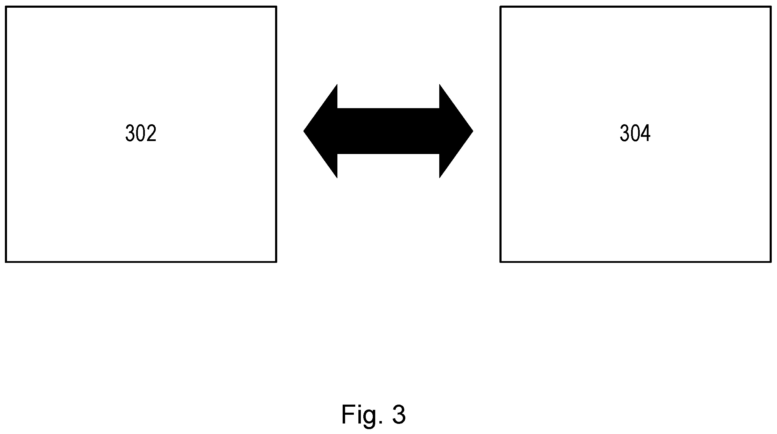

Embodiments of the present invention also provide a computer program product. Fig. 3 is a schematic illustration of an example of a processor 302 in communication with a computer-readable medium 304. A computer program product comprises a non-transitory computer-readable medium 304, the computer-readable medium having computer-readable code embodied therein, the computer-readable code being configured such that, on execution by a suitable computer or processor 302, the computer or processor is caused to perform steps of the method 100 disclosed herein.

As disclosed herein, the present invention provides a mechanism by which a subject may be assigned a priority classification (e.g., a priority score) which can be used to determine an appropriate adherence management action to be taken in respect of the subject, and an urgency with which the action should be taken. In response to determining the priority classification, embodiments of the present invention may also take the necessary action, for example initiating adherence management with the subject. By determining the priority of the subject based on medical data relating to the subject and data relating to the subject's past adherence to treatments, it is possible to more appropriately prioritize those subjects to greater need of assistance to help them adhere to treatment for a particular condition. This can help to ensure that those subjects (e.g., patients) in greatest need for adherence management assistance receive the help they need, while reducing the amount of time and resources spent assisting subjects who are lower priority.

The processor 202, 302 can comprise one or more processors, processing units, multicore processors or modules that are configured or programmed to control the apparatus 200 in the manner described herein. In particular implementations, the processor 202, 302 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g., Java interpreter instructions). Alternatively, one or more or all of the sub-routines may be stored in at least one external library file and linked with a main program either statically or dynamically, e.g., at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (100) for assessing a subject's adherence to a treatment for a condition, the method comprising:

   receiving (102) adherence data indicative of the subject's past adherence to the treatment;
   receiving (104) medical data indicative of physiological details and a medical history of the subject;
   determining (106), based on the received adherence data, a non-adherence risk score indicative of a likelihood that the subject will not adhere to the treatment within a defined time period in the future;
   determining (108), based on the medical data, an adverse event risk score indicative of a likelihood that the subject will experience an adverse medical event;
   determining (110), based on the non-adherence risk score and the adverse event risk score, a priority classification to be assigned to the subject; and
   generating (122), based on the priority classification, an instruction signal to be delivered to a recipient.

2. A computer-implemented method (100) according to claim 1, wherein generating the instruction signal comprises generating a signal to instruct the recipient to take action to improve the subject's adherence to the treatment.

3. A computer-implemented method (100) according to claim 2, wherein the signal comprises at least one of: a signal to instruct an operator to arrange a coaching session with the subject; a signal to instruct an operator to initiate contact with the subject; a signal to instruct a computing device to initiate contact with the subject; a signal to adjust an operating parameter of a treatment device.

4. A computer-implemented method (100) according to any of the preceding claims, wherein the adherence data comprises data selected from a group comprising: an indication of an average adherence to the treatment over a defined period; an indication of a number of days in a defined period when the subject did not adhere to the treatment; and an indication of a variability of adherence to the treatment over a defined period.

5. A computer-implemented method (100) according to any of the preceding claims, wherein the medical data comprises data selected from a group comprising: an age of the subject, a weight of the subject, a blood pressure of the subject, a heart rate of the subject, a breath rate of the subject, a comorbidity associated with the subject and an indication of the presence of one or more biomarkers in the subject, the biomarkers selected from a group comprising: creatinine kinase, C-reactive protein, B-type natriuretic peptide (BNP), troponin I, troponin T, and N-terminal fragment of proBNP (NT-proBNP).

6. A computer-implemented method (100) according to any of the preceding claims, further comprising:

   receiving (112) covariate data indicative of one or more covariates that affect the health of the subject; and
   determining a priority classification to be assigned to the subject further based on the covariate data.

7. A computer-implemented method (100) according to claim 6, wherein the covariate data comprises data selected from a group comprising: an indication of an expected future adherence to the treatment, an age of the subject, a gender of the subject, a race of the subject, a socio-economic class of the subject, an indication of comorbidities suffered by the subject, a blood oxygen level of the subject, and an indication of an apnea or hypopnea suffered by the subject.

8. A computer-implemented method (100) according to claims 1 to 5, wherein determining the priority classification comprises applying the non-adherence risk score and the adverse event risk score to a model of the form:

$$f\left(R_{\mathrm{prio}}\right) = g(R, Z|\beta)$$

   where $f(R_{prio})$ is a priority transformation function; $R$ is a vector of the non-adherence risk score, $R_{NA}$, and the adverse event risk score, $R_{AE}$; $Z$ is a vector of covariates that affect the health of the subject; $\beta$ is a vector of model parameters; and g is a function that calculates a score based on $R$ and $Z$ given $\beta$.

9. A computer-implemented method (100) according to claim 8, further comprising:

   receiving (120), via a user input, an indication of an adjustment to be made to at least one parameter in the vector of model parameters; and
   determining a priority classification based on the adjusted vector of model parameters.

10. A computer-implemented method (100) according to claims 1 to 5, wherein determining the priority classification comprises applying the non-adherence risk score and the adverse event risk score to a trained predictive model.

11. A computer-implemented method (100) according to claim 10, wherein the trained predictive model comprises a Cox proportional-hazards model.

12. A computer-implemented method (100) according to any of the preceding claims, further comprising:

   applying (118) weights to the non-adherence risk score and the adverse event risk score; and
   determining a priority classification based on the weighted non-adherence risk score and the weighted adverse event risk score.

13. A computer-implemented method (100) according to claims 1 to 6, further comprising:

   receiving (114) medical condition data relating to a particular medical condition from which the subject is suffering;
   determining (116), based on the medical condition data, a medical condition risk score indicative of a likelihood that the subject will suffer an adverse event related to the medical condition; and
   determining a priority classification further based on the medical condition risk score.

14. An apparatus (200) comprising:
   a processor (202) configured to perform a method according any of the preceding claims.

15. A computer program product comprising a non-transitory computer-readable medium (304), the computer-readable medium having computer-readable code embodied therein, the computer-readable code being configured such that, on execution by a suitable computer or processor (302), the computer or processor is caused to perform the method according to any of the claims 1 to 13.

Fig. 1

EP 4 345 841 A1

200

202

Fig. 2

EP 4 345 841 A1

302    ⟷    304

Fig. 3

Europäisches
Patentamt

European
Patent Office

Office européen
des brevets

# EUROPEAN SEARCH REPORT

Application Number

EP 22 20 2812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/113985 A1 (GANDY KIMBERLY L [US] ET AL) 26 April 2018 (2018-04-26) * paragraph [0004] – paragraph [0008] * * paragraph [0026] – paragraph [0033] * * paragraph [0045] – paragraph [0052] * ----- | 1-15 | INV. G16H50/70 G16H20/00 G16H50/30 |
| A | SWEED RANIA AHMAD ET AL: "Comorbidities associated with obstructive sleep apnea: a retrospective Egyptian study on 244 patients", SLEEP AND BREATHING - SCHLAF UND ATMUNG, DRUCKBILD, TITISEE-NEUSTADT, DE, vol. 23, no. 4, 26 January 2019 (2019-01-26), pages 1079-1085, XP036948829, ISSN: 1520-9512, DOI: 10.1007/S11325-019-01783-W [retrieved on 2019-01-26] * the whole document * ----- | 1-15 | |

TECHNICAL FIELDS
SEARCHED       (IPC)

G16H

The present search report has been drawn up for all claims

| Place of search | Date of completion of the search | Examiner |
|---|---|---|
| Munich | 11 July 2023 | Gardiner, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 2812

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2023

| Patent document cited in search report | Publication date | Patent family member(s) | Publication date |
|---|---|---|---|
| US 2018113985 A1 | 26-04-2018 | NONE | |

For more details about this annex : see Official Journal of the European Patent Office, No. 12/82